# EUROPEAN PATENT SPECIFICATION

(11) **EP 1 386 624 B1**
(45) Date of publication and mention of the grant of the patent: **27.05.2009**
(21) Application number: 03254747.3
(22) Date of filing: 29.07.2003
(51) Int. Cl.: A61L 31/16, A61L 31/10

(54) **Coated vascular devices**
Beschichtete Gefässvorrichtungen
Dispositifs vasculaires présentant un revêtement

(30) Priority: 30.07.2002 US 208581
(43) Date of publication of application: 04.02.2004
(73) Proprietor: Cordis Corporation, Miami Lakes, Florida 33014 (US)
(72) Inventor: Bosma, Gjalt, 9218 PV Opeinde (NL); Van Der Meulen, Joost, 9251 PD Bergum (NL)
(74) Representative: Belcher, Simon James

(56) References cited:
- EP-A- 0 815 803
- EP-A- 0 968 688
- EP-A- 1 192 957
- WO-A-01/87376
- WO-A-02/26139
- WO-A-02/26281

## Description

### CROSS REFERENCE TO RELATED APPLICATIONS

This application is a continuation-in-part application of U.S. Application Serial Number 10/136,569 filed April 30, 2002.

### BACKGROUND OF THE INVENTION

### 1. Field of the Invention

The present invention relates to the local administration of drug/drug combinations for the prevention and treatment of vascular disease, and more particularly to intraluminal medical devices for the local delivery of drug/drug combinations for the prevention and treatment of vascular disease caused by injury and methods for maintaining the drug/drug combinations on the intraluminal medical devices. The present invention also relates to medical devices having drugs, agents and/or compounds affixed thereto to treat and prevent disease and minimize or substantially eliminate a biological organism's reaction to the introduction of the medical device to the organism. In addition, the drugs, agents and/or compounds may be utilized to promote healing.

### 2. Discussion of the Related Art

Many individuals suffer from circulatory disease caused by a progressive blockage of the blood vessels that profuse the heart and other major organs. More severe blockage of blood vessels in such individuals often leads to hypertension, ischemic injury, stroke, or myocardial infarction. Atherosclerotic lesions, which limit or obstruct coronary blood flow, are the major cause of ischemic heart disease. Percutaneous transluminal coronary angioplasty is a medical procedure whose purpose is to increase blood flow through an artery. Percutaneous transluminal coronary angioplasty is the predominant treatment for coronary vessel stenosis. The increasing use of this procedure is attributable to its relatively high success rate and its minimal invasiveness compared with coronary bypass surgery. A limitation associated with percutaneous transluminal coronary angioplasty is the abrupt closure of the vessel which may occur immediately after the procedure and restenosis which occurs gradually following the procedure. Additionally, restenosis is a chronic problem in patients who have undergone saphenous vein bypass grafting. The mechanism of acute occlusion appears to involve several factors and may result from vascular recoil with resultant closure of the artery and/or deposition of blood platelets and fibrin along the damaged length of the newly opened blood vessel.

Restenosis after percutaneous transluminal coronary angioplasty is a more gradual process initiated by vascular injury. Multiple processes, including thrombosis, inflammation, growth factor and cytokine release, cell proliferation, cell migration and extracellular matrix synthesis each contribute to the restenotic process.

While the exact mechanism of restenosis is not completely understood, the general aspects of the restenosis process have been identified. In the normal arterial wall, smooth muscle cells proliferate at a low rate, approximately less than 0.1 percent per day. Smooth muscle cells in the vessel walls exist in a contractile phenotype characterized by eighty to ninety percent of the cell cytoplasmic volume occupied with the contractile apparatus. Endoplasmic reticulum, Golgi, and free ribosomes are few and are located in the perinuclear region. Extracellular matrix surrounds the smooth muscle cells and is rich in heparin-like glycosylaminoglycans which are believed to be responsible for maintaining smooth muscle cells in the contractile phenotypic state (Campbell and Campbell, 1985).

Upon pressure expansion of an intracoronary balloon catheter during angioplasty, smooth muscle cells within the vessel wall become injured, initiating a thrombotic and inflammatory response. Cell derived growth factors such as platelet derived growth factor, basic fibroblast growth factor, epidermal growth factor, thrombin, etc., released from platelets, invading macrophages and/or leukocytes, or directly from the smooth muscle cells provoke a proliferative and migratory response in medial smooth muscle cells. These cells undergo a change from the contractile phenotype to a synthetic phenotype characterized by only a few contractile filament bundles, extensive rough endoplasmic reticulum, Golgi and free ribosomes. Proliferation/migration usually begins within one to two days post-injury and peaks several days thereafter (Campbell and Campbell, 1987; Clowes and Schwartz, 1985).

Daughter cells migrate to the intimal layer of arterial smooth muscle and continue to proliferate and secrete significant amounts of extracellular matrix proteins. Proliferation, migration and extracellular matrix synthesis continue until the damaged endothelial layer is repaired at which time proliferation slows within the intima, usually within seven to fourteen days post-injury. The newly formed tissue is called neointima. The further vascular narrowing that occurs over the next three to six months is due primarily to negative or constrictive remodeling.

Simultaneous with local proliferation and migration, inflammatory cells adhere to the site of vascular injury. Within three to seven days post-injury, inflammatory cells have migrated to the deeper layers of the vessel wall. In animal models employing either balloon injury or stent implantation, inflammatory cells may persist at the site of vascular injury for at least thirty days (Tanaka et al., 1993; Edelman et al., 1998). Inflammatory cells therefore are present and may contribute to both the acute and chronic phases of restenosis.

Numerous agents have been examined for presumed anti-proliferative actions in restenosis and have shown some activity in experimental animal models. Some of the agents which have been shown to successfully reduce the extent of intimal hyperplasia in animal models include: heparin and heparin fragments (Clowes, A.W. and Karnovsky M., Nature 265: 25-26, 1977; Guyton, J.R. et al., Circ. Res., 46: 625-634, 1980; Clowes, A.W. and Clowes, M.M., Lab. Invest. 52: 611-616, 1985; Clowes, A.W. and Clowes, M.M., Circ. Res. 58: 839-845, 1986; Majesky et al., Circ. Res. 61: 296-300, 1987; Snow et al., Am. J. Pathol. 137: 313-330, 1990; Okada, T. et al., Neurosurgery 25: 92-98, 1989), colchicine (Currier, J.W. et al., Circ. 80: 11-66, 1989), taxol (Sollot, S.J. et al., J. Clin. Invest. 95: 1869-1876, 1995), angiotensin converting enzyme (ACE) inhibitors (Powell, J.S. et al., Science, 245: 186-188, 1989), angiopeptin (Lundergan, C.F. et al. Am. J. Cardiol. 17(Suppl. B):132B-136B, 1991), cyclosporin A (Jonasson, L. et al., Proc. Natl., Acad. Sci., 85: 2303, 1988), goat-anti-rabbit PDGF antibody (Fems, G.A.A., et al., Science 253: 1129-1132, 1991), terbinafine (Nemecek, G.M. et al., J. Pharmacol. Exp. Thera. 248: 1167-1174, 1989), trapidil (Liu, M.W. et al., Circ. 81: 1089-1093, 1990), tranilast (Fukuyama, J. et al., Eur. J. Pharmacol. 318: 327-332, 1996), interferon-gamma (Hansson, G.K. and Holm, J., Circ. 84: 1266-1272, 1991), rapamycin (Marx, S.O. et al., Circ. Res. 76: 412-417, 1995), steroids (Colburn, M.D. et al., J. Vasc. Surg. 15: 510-518, 1992), see also Berk, B.C. et al., J. Am. Coll. Cardiol. 17: 111B-117B, 1991), ionizing radiation (Weinberger, J. et al., Int. J. Rad. Onc. Biol. Phys. 36: 767-775, 1996), fusion toxins (Farb, A. et al., Circ. Res. 80: 542-550, 1997) antisense oligionucleotides (Simons, M. et al., Nature 359: 67-70, 1992) and gene vectors (Chang, M.W. et al., J. Clin. Invest. 96: 2260-2268, 1995). Anti-proliferative action on smooth muscle cells *in vitro* has been demonstrated for many of these agents, including heparin and heparin conjugates, taxol, tranilast, colchicine, ACE inhibitors, fusion toxins, antisense oligionucleotides, rapamycin and ionizing radiation. Thus, agents with diverse mechanisms of smooth muscle cell inhibition may have therapeutic utility in reducing intimal hyperplasia.

However, in contrast to animal models, attempts in human angioplasty patients to prevent restenosis by systemic pharmacologic means have thus far been unsuccessful. Neither aspirin-dipyridamole, ticlopidine, anti-coagulant therapy (acute heparin, chronic warfarin, hirudin or hirulog), thromboxane receptor antagonism nor steroids have been effective in preventing restenosis, although platelet inhibitors have been effective in preventing acute reocclusion after angioplasty (Mak and Topol, 1997; Lang et al., 1991; Popma et al., 1991). The platelet GP II_{b}/IIIₐ receptor, antagonist, Reopro® is still under study but Reopro® has not shown definitive results for the reduction in restenosis following angioplasty and stenting. Other agents, which have also been unsuccessful in the prevention of restenosis, include the calcium channel antagonists, prostacyclin mimetics, angiotensin converting enzyme inhibitors, serotonin receptor antagonists, and anti-proliferative agents. These agents must be given systemically, however, and attainment of a therapeutically effective dose may not be possible; anti-proliferative (or anti-restenosis) concentrations may exceed the known toxic concentrations of these agents so that levels sufficient to produce smooth muscle inhibition may not be reached (Mak and Topol, 1997; Lang et al., 1991; Popma et al., 1991).

Additional clinical trials in which the effectiveness for preventing restenosis utilizing dietary fish oil supplements or cholesterol lowering agents has been examined showing either conflicting or negative results so that no pharmacological agents are as yet clinically available to prevent post-angioplasty restenosis (Mak and Topol, 1997; Franklin and Faxon, 1993: Serruys, P.W. et al., 1993). Recent observations suggest that the antilipid/antioxident agent, probucol, may be useful in preventing restenosis but this work requires confirmation (Tardif et al., 1997; Yokoi, et al., 1997). Probucol is presently not approved for use in the United States and a thirty-day pretreatment period would preclude its use in emergency angioplasty. Additionally, the application of ionizing radiation has shown significant promise in reducing or preventing restenosis after angioplasty in patients with stents (Teirstein et al., 1997). Currently, however, the most effective treatments for restenosis are repeat angioplasty, atherectomy or coronary artery bypass grafting, because no therapeutic agents currently have Food and Drug Administration approval for use for the prevention of post-angioplasty restenosis.

Unlike systemic pharmacologic therapy, stents have proven useful in significantly reducing restenosis. Typically, stents are balloon-expandable slotted metal tubes (usually, but not limited to, stainless steel), which, when expanded within the lumen of an angioplastied coronary artery, provide structural support through rigid scaffolding to the arterial wall. This support is helpful in maintaining vessel lumen patency. In two randomized clinical trials, stents increased angiographic success after percutaneous transluminal coronary angioplasty, by increasing minimal lumen diameter and reducing, but not eliminating, the incidence of restenosis at six months (Serruys et al., 1994; Fischman et al., 1994).

Additionally, the heparin coating of stents appears to have the added benefit of producing a reduction in sub-acute thrombosis after stent implantation (Serruys et al., 1996). Thus, sustained mechanical expansion of a stenosed coronary artery with a stent has been shown to provide some measure of restenosis prevention, and the coating of stents with heparin has demonstrated both the feasibility and the clinical usefulness of delivering drugs locally, at the site of injured tissue.

As stated above, the use of heparin coated stents demonstrates the feasibility and clinical usefulness of local drug delivery; however, the manner in which the particular drug or drug combination is affixed to the local delivery device will play a role in the efficacy of this type of treatment. For example, the processes and materials utilized to affix the drug/drug combinations to the local delivery device should not interfere with the operations of the drug/drug combinations. In addition, the processes and materials utilized should be biocompatible and maintain the drug/drug combinations on the local device through delivery and over a given period of time. For example, removal of the drug/drug combination during delivery of the local delivery device may potentially cause failure of the device.

Accordingly, there exists a need for drug/drug combinations and associated local delivery devices for the prevention and treatment of vascular injury causing intimal thickening which is either biologically induced, for example, atherosclerosis, or mechanically induced, for example, through percutaneous transluminal coronary angioplasty. In addition, there exists a need for maintaining the drug/drug combinations on the local delivery device through delivery and positioning as well as ensuring that the drug/drug combination is released in therapeutic dosages over a given period of time.

A variety of stent coatings and compositions have been proposed for the prevention and treatment of injury causing intimal thickening. The coatings may be capable themselves of reducing the stimulus the stent provides to the injured lumen wall, thus reducing the tendency towards thrombosis or restenosis. Alternately, the coating may deliver a pharmaceutical/therapeutic agent or drug to the lumen that reduces smooth muscle tissue proliferation or restenosis. The mechanism for delivery of the agent is through diffusion of the agent through either a bulk polymer or through pores that are created in the polymer structure, or by erosion of a biodegradable coating.

Both bioabsorbable and biostable compositions have been reported as coatings for stents. They generally have been polymeric coatings that either encapsulate a pharmaceutical/therapeutic agent or drug, e.g. rapamycin, taxol etc., or bind such an agent to the surface, e.g. heparin-coated stents. These coatings are applied to the stent in a number of ways, including, though not limited to, dip, spray, or spin coating processes.

One class of biostable materials that has been reported as coatings for stents is polyfluoro homopolymers. Polytetrafluoroethylene (PTFE) homopolymers have been used as implants for many years. These homopolymers are not soluble in any solvent at reasonable temperatures and therefore are difficult to coat onto small medical devices while maintaining important features of the devices (e.g. slots in stents).

Stents with coatings made from polyvinylidenefluoride homopolymers and containing pharmaceutical/therapeutic agents or drugs for release have been suggested. However, like most crystalline polyfluoro homopolymers, they are difficult to apply as high quality films onto surfaces without subjecting them to relatively high temperatures, that correspond to the melting temperature of the polymer.

It would be advantageous to develop coatings for implantable medical devices that will reduce thrombosis, restenosis, or other adverse reactions, that may include, but do not require, the use of pharmaceutical or therapeutic agents or drugs to achieve such affects, and that possess physical and mechanical properties effective for use in such devices even when such coated devices are subjected to relatively low maximum temperatures. It would also be advantageous to develop implantable medical devices in combination with various drugs, agents and/or compounds which treat disease and minimize or substantially eliminate a living organisms' reaction to the implantation of the medical device.

### SUMMARY OF THE INVENTION

The drug/drug combination therapies, drug/drug combination carriers and associated local delivery devices of the present invention provide a means for overcoming the difficulties associated with the methods and devices currently in use, as briefly described above. In addition, the methods for maintaining the drug/drug combination therapies, drug/drug combination carriers on the local delivery device ensure that the drug/drug combination therapies reach the target site.

In accordance with present invention is directed to a removable percutaneous vascular filter system for blocking micro- and macro-emboli in a vessel while allowing continued perfusion of blood. The removable percutaneous vascular filter system comprises a guidewire having distal and proximal ends, a filter including a non-metallic, porous, flexible filter membrane having a distal portion and a proximal free portion and a filter membrane support structure extending from the flexible filter membrane distal portion to at least the flexible filter membrane proximal portion, wherein the filter membrane distal portion in pivotally attached to the guidewire near the distal end of the guidewire and wherein the filter membrane proximal free end portion is substantially parallel to the guidewire in its collapsed state, deploying means operatively connected to the filter to cause the filter membrane proximal free end portion to move from a position substantially parallel to the guidewire to a position removed from the longitudinal axis of the guidewire to cause the flexible filter membrane to form a substantially conical shape to form a generally sealing relationship with the wall of the vessel, a biocompatible vehicle affixed to at least a portion of the filter, and at least one agent in therapeutic dosages incorporated into the biocompatible vehicle for the treatment of a disease condition.

The medical devices, drug coatings and methods for maintaining the drug coatings or vehicles thereon of the present invention utilizes a combination of materials to treat disease, and reactions by living organisms due to the implantation of medical devices for the treatment of disease or other conditions. The local delivery of drugs, agents or compounds generally substantially reduces the potential toxicity of the drugs, agents or compounds when compared to systemic delivery while increasing their efficacy.

Drugs, agents or compounds may be affixed to any number of medical devices to treat various diseases. The drugs, agents or compounds may also be affixed to minimize or substantially eliminate the biological organism's reaction to the introduction of the medical device utilized to treat a separate condition. For example, stents may be introduced to open coronary arteries or other body lumens such as biliary ducts. The introduction of these stents cause a smooth muscle cell proliferation effect as well as inflammation. Accordingly, the stents may be coated with drugs, agents or compounds to combat these reactions. Anastomosis devices, routinely utilized in certain types of surgery, may also cause a smooth muscle cell proliferation effect as well as inflammation. Stent-grafts and systems utilizing stent-grafts, for example, aneurysm bypass systems may be coated with drugs, agents and/or compounds which prevent adverse affects caused by the introduction of these devices as well as to promote healing and incorporation. Therefore, the devices may also be coated with drugs, agents and/or compounds to combat these reactions.

The drugs, agents or compounds will vary depending upon the type of medical device, the reaction to the introduction of the medical device and/or the disease sought to be treated. The type of coating or vehicle utilized to immobilize the drugs, agents or compounds to the medical device may also vary depending on a number of factors, including the type of medical device, the type of drug, agent or compound and the rate of release thereof.

In order to be effective, the drugs, agents or compounds should preferably remain on the medical devices during delivery and implantation. Accordingly, various coating techniques for creating strong bonds between the drugs, agents or compounds may be utilized. In addition, various materials may be utilized as surface modifications to prevent the drugs, agents or compounds from coming off prematurely.

### BRIEF DESCRIPTION OF THE DRAWINGS

The foregoing and other features and advantages of the invention will be apparent from the following, more particular description of preferred embodiments of the invention, as illustrated in the accompanying drawings.
Figure 30 is a perspective view of a vascular filter in a position of use and also a delivery device.
Figures 31 and 32 are schematic representations of the various stages of manufacture of the vascular filter of Figure 30.
Figure 33 is a perspective view of another exemplary embodiment of a vascular filter in accordance with the present invention.
Figure 34 is a lateral, partly cross-sectional view of the distal end of a guidewire of one exemplary embodiment of a vascular filter with the filter membrane in a collapsed position in accordance with the present invention.
Figure 35 is a lateral, party cross-sectional view of the distal end of the guidewire of Figure 34 with the filter membrane in an expanded or deployed position.
Figure 36 is a proximal end-on view of the filter membrane illustrated in Figure 35.

### DETAILED DESCRIPTION OF THE PREFERRED EMBODIMENTS

Percutaneous transluminal coronary angioplasty (PTCA), stenting, and atherectomy are therapeutic medical procedures used to increase blood flow through the coronary arteries. These procedures may often be performed as alternatives to coronary bypass surgery. Percutaneous transluminal angioplasty (PTA) and stenting may often be performed as alternatives to carotid endarterectomy, and femoral-popliteal bypass procedures. In PTCA or PTA procedures, the angioplasty balloon is inflated within the stenosed vessel, at the location of an occlusion, in order to shear and disrupt the wall components of the vessel to obtain an enlarged lumen. In stenting, an endoluminal prosthesis is implanted in the vessel to maintain patency following the procedure. In atherectomy, a rotating blade is used to shear plaque from the arterial wall.

One of the potential complications associated with all of these techniques is the accidental dislodgment of plaque, thrombus or other embolic particulates generated during manipulation of the vessel, thereby potentially causing occlusion of the narrower vessels downstream, which may lead to ischemia or infarct of the organ which the vessel supplies. Such emboli may be extremely dangerous to the patient, and may result in myocardial infarction, stroke or limb ischemia. In 1995, Waksman et al. disclosed that distal embolization is common after directional atherectomy in coronary arteries and saphenous vein grafts. See Waksman et al., American Heart Journal 129(3): 430-5(1995). This study found that distal embolization occurs in twenty-eight percent (31 out of 111) of the patients undergoing atherectomy. In January 1999, Jordan, Jr. et al. disclosed that the treatment of carotid stenosis using percutaneous angioplasty with stenting procedure is associated with more than eight times the rate of micro-emboli seen using carotid endarterectomy. See Jordan, Jr. et al. Cardiovascular Surgery 7(1): 33-8 (1999). Micro-emboli, as detected by transcranial Doppler monitoring in this study, have been shown to be a potential cause of stroke. The embolic materials include calcium, intimal debris, atheromatous plaque, and thrombi.

Another area in which debris may cause a problem is in coronary bypass surgery. In conventional bypass surgery, the aorta is clamped to stop blood flow. If the aorta is diseased, debris may be released when the clamp is removed and carried away downstream. As described above, this debris may then block off the blood to other organs, including the brain.

Vascular filters are well known in the art, especially vena cava filters, as illustrated in U.S. Patent Nos. 4,727,873 and 4,688,553. There is also a substantial amount of medical literature describing various designs of vascular filters and reporting the results of clinical and experimental use thereof. See, for example, the article by Eichelter and Schenk, entitled "Prophylaxis of Pulmonary Embolism," Archives of Surgery, Vol. 97 (August, 1968). See, also, the article by Greenfield, et al. entitled "A New Intracaval Filter Permitting Continued Flow and Resolution of Emboli," Surgery, Vol. 73, No. 4 (1973).

Vascular filters are often used during a postoperative period, when there is a perceived risk of a patient encountering pulmonary embolism resulting from clots generated peri-operatively. Pulmonary embolism is a serious and potentially fatal condition that occurs when these clots travel to the lungs. The filter is therefore typically placed in the vena cava to catch and trap clots before they can reach the lungs.

Many of the vascular filters in the prior art are intended to be permanently placed in the venous system of the patient, so that even after the need for the filter has passed, the filter remains in place for the life of the patient. U.S. Patent No. 3,952,747 describes a stainless steel filtering device that is permanently implanted transvenously within the inferior vena cava. This device is intended to treat recurrent pulmonary embolism. Permanent implantation is often deemed medically undesirable, but it is done because filters are implanted in patients in response to potentially life-threatening situations.

To avoid permanent implantation, it is highly desirable to provide an apparatus and method for preventing embolization associated with angioplasty, stenting or other procedures. In particular, it is desirable to provide a device that may be temporarily placed within the vascular system to collect and retrieve plaque, thrombus and other embolic particulates that have been dislodged during angioplasty, stenting or other procedures. Such a device is removed at the end of the procedure. U.S. Patent Nos. 6,179,861 and 6,001,118 describe guidewire-based filters where the filter resembles a windsock and is supported by one or more articulated support hoops. U.S. Patent Nos. 5,814,064 and 5,827,324 describe guidewire-based filter devices, wherein the filter is expanded to a predetermined diameter through the introduction of a fluid or a gas. U.S. Patent Nos. 6,168,604 and 6,152,946 describe guidewire-based filters, wherein the diameter of the filter is controlled by advancing and retracting a sheath over the filter component.

Accordingly, vascular filters may be permanently implantable or temporarily placed within the vascular system to collect and retrieve plaque, thrombus and other embolic particulates that have become dislodged. There are a wide variety of designs, configurations and materials for vascular filters depending on the particular application. Similarly to stents, anastomosis devices, stent-grafts and other medical devices, vascular filters may cause some injury to the target vessel, thereby provoking a response from the body. Therefore, as in the case with stents, anastomosis devices and stent-grafts, there is the potential for smooth muscle cell proliferation, localized inflammation as well as other conditions. Accordingly, there is a need to minimize or substantially eliminate smooth muscle cell proliferation and inflammation at the placement site of the vascular filter as well as other potential adverse reactions. Rapamycin and/or other drugs, agents or compounds may be used in a manner analogous to the stents as described above. In other words, at least a portion of the vascular filter may be coated with rapamycin or other drugs, agents and/or compounds such as heparin for reducing the risk of thrombosis.

Although there are many different types of vascular filters that may be coated with rapamycin and/or other drugs, agents and/or compounds, the particular exemplary embodiments set forth below are directed to vena cava filters.

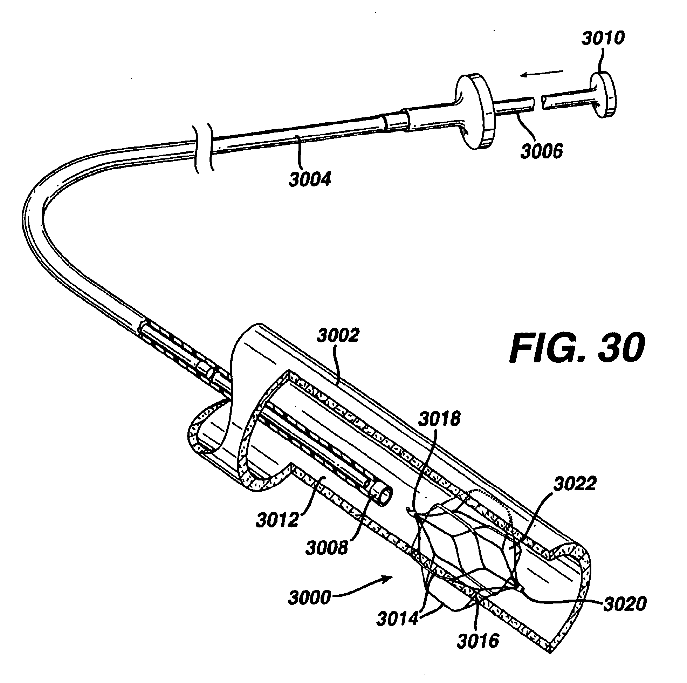

Referring to Figure 30, there is illustrated a first embodiment of a vena cava filter 3000 by way of background understanding. The vena cava filter 3000 may be introduced into the lumpen of a blood vessel 3002 percutaneously via a delivery catheter 3004. In one exemplary embodiment, the vena cava filter 3000 is ejected from the delivery catheter 3004 by means of a pushing wire 3006. The pushing wire 3006 simply forces the vena cava filter 3000 from the distal tip 3008 of the delivery catheter 3004 when the physician moves the proximal end 3010 of the pushing wire 3006 in the direction indicated by the arrow. Once the vena cava filter 3000 is ejected from the distal tip 3008, the vena cava filter 3000 expands, under the influence of expansive forces inherent to the material from which the vena cava filter 3000 is constructed, into the shape illustrated. The vena cava filter 3000 is designed to make contact with and engage the inner wall 3012 of the blood vessel 3002. It is preferable that the vena cava filter 3000 engage the inner wall 3012 of the blood vessel 3002 such that it does not move once positioned.

The vena cava filter 3000 comprises a plurality of longitudinally arranged first elements 3014, which when the vena cava filter 3000 is fully expanded, make engaging contact with the inner wall 3012 of the blood vessel 3002. As is explained above generally, and in more detail below, the vena cava filter 3000 comprises a material which may be programmed to exert a chronic outward force at body temperature. The vena cava filter 3000 also comprises a plurality of second elements 3016 connected to the plurality of first elements 3014 and to each other to form a symmetrical, multi-faceted, elongated, substantially tubular body member. The second elements 3016 extend from the plurality of first elements 3014 towards the longitudinal axis of the substantially tubular body member where they are connected together to form tubular end portions 3018 and 3020. As illustrated in Figure 30, the multi-faceted, elongated, substantially tubular body member that defines the vena cava filter 3000 is symmetrical, which as explained below provides for a number of advantages over existing vena cava filters. The plurality of first elements 3014, the plurality of second elements 3016 and the tubular end portions 3018 and 3020 may be formed from separate components which may be joined in any number of ways, or more preferably from a single element such as a tubular member machined and heat treated into the various components described above.

The grids or lattice structures formed by the plurality of interconnected second elements 3016 comprise the filter sections 3022 of the vena cava filter 3000. Since the substantially tubular body comprising the vena cava filter 3000 is symmetrical, there are essentially two sets of filter sections 3022 on either side of the plurality of first elements 3014. The filter sections 3022 or spaces between the plurality of interconnected second elements 3016 are sized to allow blood to pass through substantially unimpeded, but thrombus and other larger particulate matter are intercepted thereby.

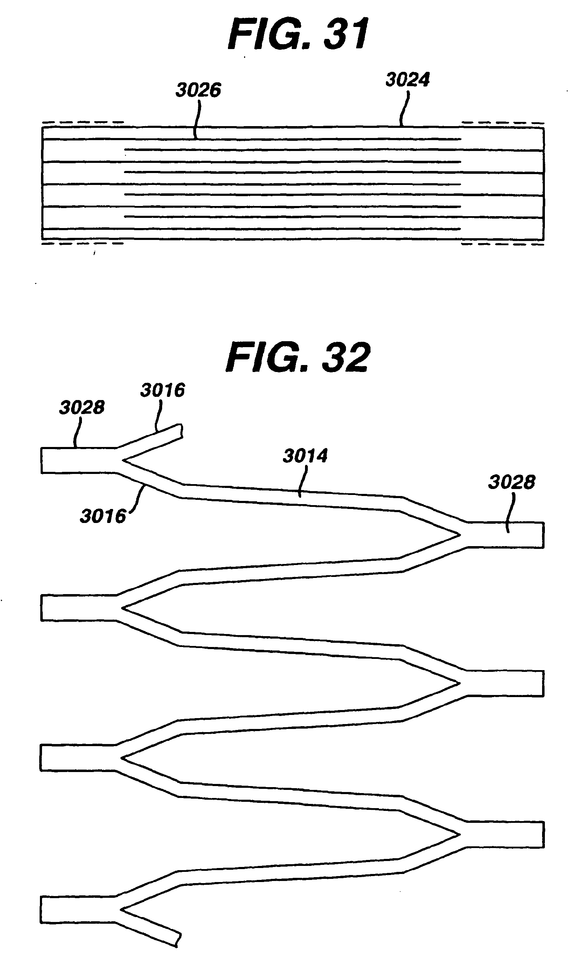

Figure 31 illustrates a plate 3024 from which the first exemplary embodiment of the vena cava filter 3000 may be constructed. The plate 3024 may be formed from any number of suitable biocompatible materials exhibiting the requisite strength requirements. In the illustrated exemplary embodiment, the plate 3024 comprises a nickel-titanium alloy. The plate 3024 is machined such that it comprises a plurality of cuts 3026. The cuts 3026 extend alternately from one of the two sides of the plate 3024 over a large portion of the plate 3024. When manufacturing a vena cava filter 3000 in accordance with the present invention, the plate 3024 comprising a plurality of cuts 3026, may be pulled apart under the influence of heat and deforming forces such that a predetermined shape, illustrated in detail in Figure 32, is obtained. As illustrated in Figure 32, the cuts 3026 in the plate 3024 result in the plurality of longitudinally arranged first elements 3014 and the plurality of second elements 3016. This drawn out planar configuration is then arranged around a mold or mandrel with a substantially circular cross section to provide the vena cava filter 3000 with a substantially tubular configuration as described above. With the planar configuration, illustrated in Figure 32, arranged around the mold or mandrel, which has the basic configuration of an elongated oval, the ends 3028, formed at the union of two second elements 3016, are connected or joined together by any number of means, including welding the ends 3028 together. The mold or mandrel allows the plurality of first elements 3014 to remain substantially parallel, while the plurality of second elements 3016 and the ends thereof 3028 are allowed to be joined together to form a solid geometric structure. The connected ends 3028 form the tubular end portions 3018 and 3020. Once the ends 3028 are joined together, the mold is expanded and the metal structure is maintained at a predetermined temperature, to deform the metal structure into the shape of the fully expanded vena cava filter 3000 illustrated in Figure 30. After cooling, the vena cava filter 3000 thus formed remains the shape of the expanded mold. It is then possible to fold or collapse the vena cava filter 3000 in order to position it within the distal tip 3008 of the catheter 3004 for the purpose of introduction into the appropriate vessel as illustrated in Figure 30.

Utilizing a nickel-titanium alloy enables the vena cava filter 3000 to be folded so as to occupy only a limited space in the distal tip 3008 of the delivery catheter 3004 and then expand independently to the diameter of the blood vessel at body temperature after introducing it in the manner described above. The super elastic qualities of nickel-titanium alloys are known in the art.

The design of the vena cava filter 3000 described and illustrated offer a number of advantages over existing vena cava filters. For example, the vena cava filter 3000 of the present invention comprises two sets of filters as described above. The two sets of filters provide two chances of intercepting thrombus moving inside the blood vessel. The design of the vena cava filter 3000 is such that only a minimum of damage to the blood vessel is incurred. Essentially, the design includes no free ends of any of the plurality of first elements 3014 and/or the plurality of second elements 3016, which may cause damage to the vessel in which it is positioned. The symmetrical design of the vena cava filter 3000 allows it to be deployed with either end placed forward or backward inside the blood vessel 3002. In addition, tipping of the vena cava filter 3000 has been avoided due to the more or less tubular shape of the plurality of first elements 3014; accordingly, the positioning of the vena cava filter 3000 inside the blood vessel 3002 may take place with unprecedented stability and reliability.

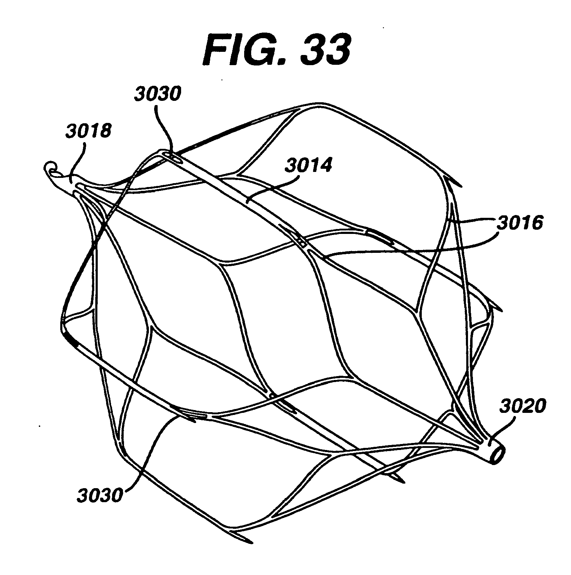

Figure 33 illustrates another exemplary embodiment of the vena cava filter 3000 in accordance with the present invention. In this exemplary embodiment, one or more of the plurality of first elements 3014 comprise hooking elements 3030 pointing in opposite directions. These hooking elements 3030 may be sufficiently sized so that they ensure the proper grip on the internal wall of the blood vessel, but tend not to damage it either. The hooking elements 3030 may be simply formed by machining notches in the plurality of first elements 3014.

Any or all of the components comprising the above-described exemplary vena cava filter 3000 may be coated with rapamycin or any other drug, agent and/or compound.

It is important to note that the above-described vena cava filter is one example of a vascular filter. Any number of vascular filters, including temporarily positioned vascular filters, may be coated with the appropriate drugs, agents and/or compounds, as well as combinations thereof. In addition, the vascular filters may be coated with coatings that comprise drugs, agents or compounds or simply with coatings that contain no drugs, agents or compounds. The entire filter may be coated or only a portion of the filter may be coated. The coating may be uniform or non-uniform. The coating may be discontinuous. The particular polymer(s) utilized for the coating depends on the particular material upon which it is affixed. In addition, the particular drug, agent and/or compound may also affect the selection of polymer(s).

Another type of vascular filter which may be used in accordance with the present invention is a distal protection device or filter. This type of filter may be used in percutaneous angioplasty and stenting procedures for the prevention of distal embolism. This type of vascular filter system allows for distal perfusion while preventing embolization. There are a wide variety of distal protection vascular filters. One particular exemplary embodiment is described below.

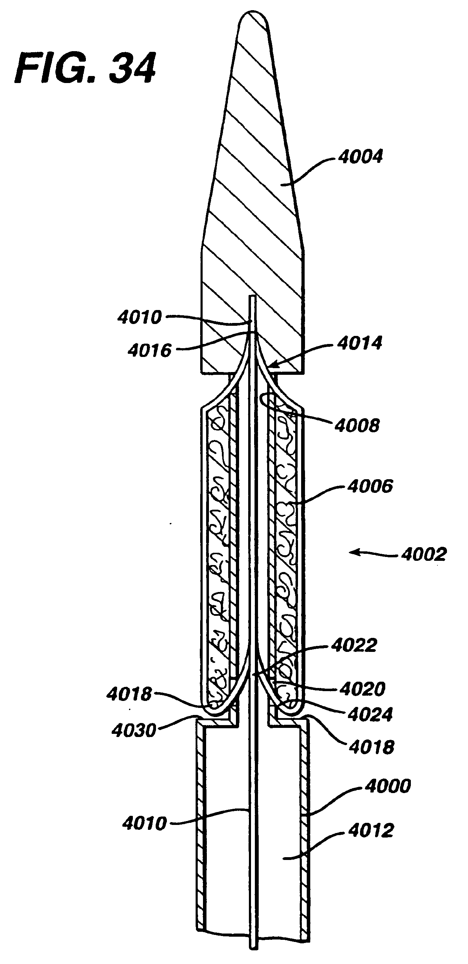

Figure 34 illustrates a lateral, cross-sectional view of a distal end of a guidewire 4000 with a filter 4002 attached thereto. Figure 34 illustrates guidewire 4000 with a shapeable, tapered soft tip 4004 at its extreme distal end which provides flexibility and maneuverability to the guidewire 4000. The filter 4002 is illustrated in a collapsed position. The filter 4002 comprises a filter membrane 4006 and a fixed portion 4008 which is movably attached to the guidewire 4000. The filter membrane 4006 lies adjacent to the guidewire 4000 proximal to the fixed portion 4008 when the filter membrane 4006 is in the collapsed state. A moveable core 4010 runs through a lumen 4012 of the guidewire 4000 and preferably extends distally a short distance beyond the fixed portion 4008 of the filter 4002. Deploying wires or fibers 4014 are each firmly attached at a first end 4016 to the moveable core 4010 distal to the fixed portion 4008 of the filter 4002. The deploying wires or fibers 4014 are each attached at a second end to the filter membrane 4006 at attachment points 4018. Collapsing wires or fibers 4020 are each attached at a first end 4022 to the portion of the moveable core wire 4010 which is interior to the filter membrane 4006 when the filter 4002 is in the collapsed state. The collapsing wires or fibers 4020 are each attached at a second end 4024 to the filter membrane 4006 at attachment point 4018. Accordingly, collapsing wires or fibers 4020 lie interior to the filter membrane 4006 when the filter membrane 4006 is in the collapsed state.

The filter membrane 4006 is deployed when the system operator or physician pulls the movable core 4010 proximally through the interior of the guidewire 4000. Prior to retraction of the movable core 4010, deploying wires or fibers 4014 are sufficiently relaxed so as not to create any tension at the filter membrane 4006 attachment points 4018. Upon retraction of the moveable core 4010, tension is created in the deploying wires or fibers 4014.

In the exemplary embodiment, the filter 4002 comprises two to six evenly spaced deploying and collapsing wires 4014, 4020. In a more preferred embodiment, the filter 4002 comprises three or four evenly spaced deploying and collapsing wires 4014 and 4020. The deploying and collapsing wires or fibers 4014 and 4020 may be constructed from any number of flexible, medically acceptable materials, including stainless steel, nitinol, or another metal or metallic alloy or a non-metallic material such as graphite or a suitable polymer. In addition, the guidewire 4000 and the moveable core 4010 may be made from the same or similar materials, as would be appreciated by those skilled in the art. Typically, the guidewire 4000 may have an external diameter in the range from about 0.014 mm to about 0.035 mm, a wall thickness in the range from about 0.002 mm to about 0.010 mm, and a length in the range from about twenty-five cm to about three-hundred cm. Also, the movable core 4010 may have a diameter in the range from about 0.003 mm to about 0.010 mm and a length in the range from about thirty cm to about three hundred-fifty cm.

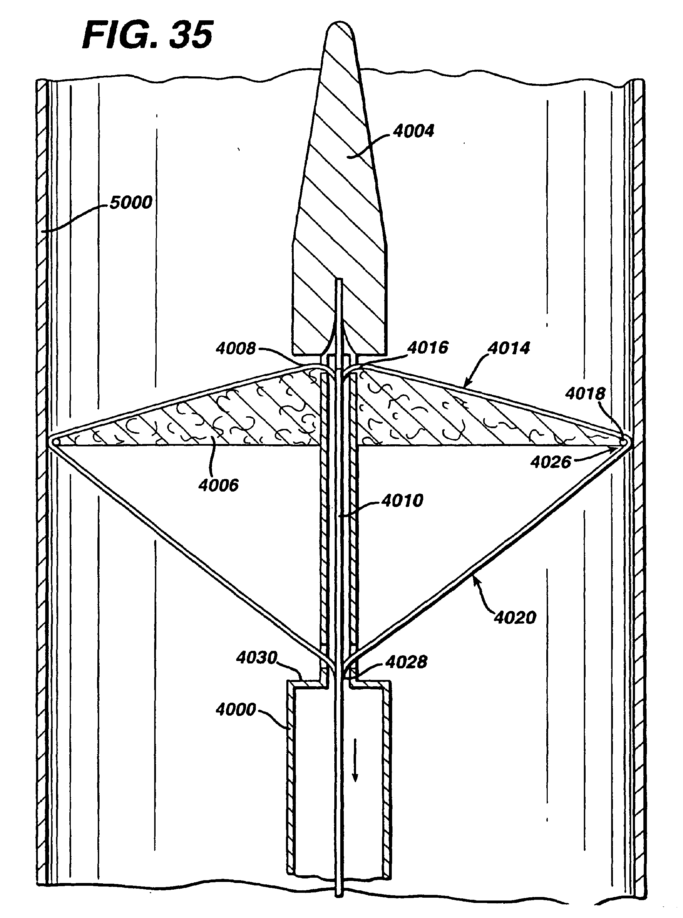

Figure 35 illustrates the exemplary guidewire 4000 and filter 4002 of the present invention deployed within an artery 5000. The movable core 4010 is in a retracted state, i.e., pulled proximally through the interior of the guidewire 4000. Tension is created in the deploying wires or fibers 4014 and the filter membrane 4006 extends to a deployed position where the outer edge 4026 of the filter membrane 4006 contacts the artery wall 5000. In this deployed position, the collapsing fibers 4020 are in a relaxed state and extend from the filter membrane attachment point 4018 to fixed attachment points 4020 on the movable core 4010.

The flow of blood through the artery 5000 is toward the distal end of the guidewire 4000. As such, the force of the blood flow exerts a force on the deployed filter membrane 4006 and helps to maintain the filter membrane 4006 in the deployed position.

For withdrawal of the guidewire 4000 and the filter 4002, the filter membrane 4006 is collapsed so that it sits tightly against the guidewire 4000. This is accomplished by extending the moveable core 4010 distally through the guidewire 4000, thereby relaxing the deploying wires or fibers 4014 and creating tension in the collapsing wires or fibers 4020. The tension in the collapsing wires or fibers 4020 collapses the filter membrane 4006, allowing it to fit tightly against the guidewire 4000 in recess 4030 as illustrated in Figure 34.

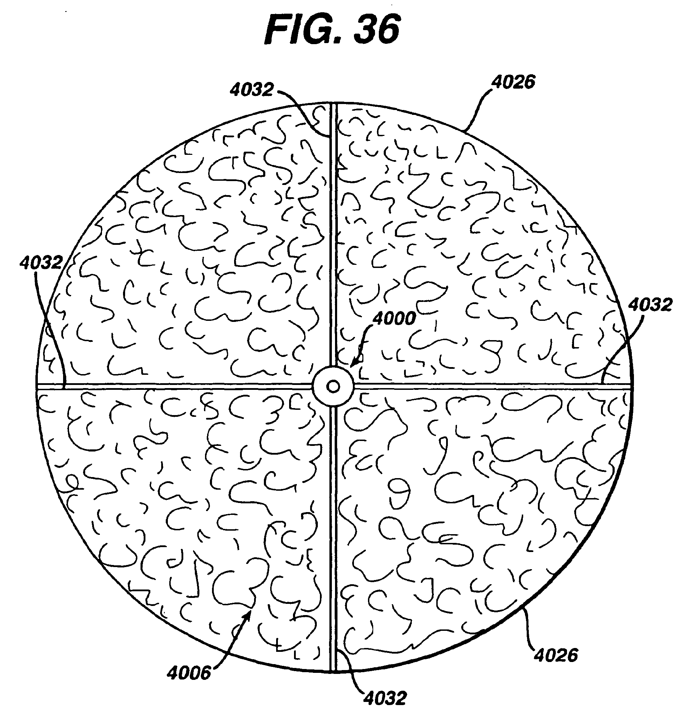

Figure 36 illustrates the filter 4002 of the present invention from a distal end view with the filter membrane 4006 deployed. The guidewire 4000 is centrally located, and structural wires 4032 extend from the guidewire 4000 to the outer edge 4020 of the filter membrane 4032. The structural wires 4032 provide structural integrity and rigidity to the filter membrane 4006. Figure 36 illustrates the filter 4002 as comprising four evenly spaced structural wires 4032; however, fewer or additional structural wires 4032 may be utilized. In a preferred embodiment, there are from two to six structural wires 4032, which may be spaced regularly or irregularly. The structural wires 4032 may comprise any number of suitable materials, including stainless steel or other medically acceptable metal or metal alloy.

The filter membrane 4006 of the invention is preferably a mesh such as illustrated in Figure 36. The mesh should preferably have pores of a size sufficient to block and capture any micro- and macro-emboli which may flow downstream from the site where the stenosis is being treated, but large enough such that blood flow is not impeded. The mesh used in the filter 4002 may have a pore size in the range from about twenty microns to about three hundred microns and preferably in the range from about fifty microns to about one hundred fifty microns. The size of the filter membrane 4006, i.e., the distance from the guidewire 4000 to the free ends 4026 is such as to allow a firm fit between the filter membrane 4006 and the artery wall 5000. Essentially, the diameter of the filter membrane 4006 will be related to the artery being treated, with typical diameters ranging from about two mm to about forty mm, and most preferably from about two mm to about twenty mm.

The filter membrane 4006 may comprise fabric or non-fabric meshes, such as those utilized in known hemodialysis filters or heart-lung bypass machine filters. Suitable materials include polymers or physiologically acceptable metals or alloys.

As is the case with the vena cava filter described above, any or all of the components comprising the above-described filter 4002 may be coated with rapamycin or any other drug, agent and/or compound. Although the above-described filter 4002 is only temporarily positioned within the body, it may be advantageous to coat the various components with one or more drugs, agents and/or compounds. For example, the components of the filter 4002, which contact the surrounding tissue, may be coated with one or more anti-proliferatives and/or one or more anti-inflammatories. These drugs may be utilized to treat any hyperproliferative or inflammatory effect that may have been caused by the temporary placement of the filter 4002. Obviously, the dosage of the therapeutic agent and the coating profile is such that the release rate of the therapeutic agent is relatively fast given the nature of the procedure. Exemplary anti-proliferatives and anti-inflammatories include rapamycin and dexamethasone. The filter membrane 4006 may be coated with these drugs or other drugs, agents and/or compounds. For example, the filter membrane 4006 may be coated with anti-coagulants, such as heparin to thin the blood in order to minimize the chance of thrombosis. The filter membrane 4006 may be coated similarly to that as the graft material discussed above.

Rapamycin is a macrocyclic triene antibiotic produced by Streptomyces hygroscopicus as disclosed in U.S. Patent No. 3,929,992. It has been found that rapamycin among other things inhibits the proliferation of vascular smooth muscle cells *in vivo*. Accordingly, rapamycin may be utilized in treating intimal smooth muscle cell hyperplasia, restenosis, and vascular occlusion in a mammal, particularly following either biologically or mechanically mediated vascular injury, or under conditions that would predispose a mammal to suffering such a vascular injury. Rapamycin functions to inhibit smooth muscle cell proliferation and does not interfere with the re-endothelialization of the vessel walls.

Rapamycin reduces vascular hyperplasia by antagonizing smooth muscle proliferation in response to mitogenic signals that are released during an angioplasty induced injury. Inhibition of growth factor and cytokine mediated smooth muscle proliferation at the late G1 phase of the cell cycle is believed to be the dominant mechanism of action of rapamycin. However, rapamycin is also known to prevent T-cell proliferation and differentiation when administered systemically. This is the basis for its immunosuppresive activity and its ability to prevent graft rejection.

As used herein, rapamycin includes rapamycin and all analogs, derivatives and congeners that bind to FKBP12, and other immunophilins and possesses the same pharmacologic properties as rapamycin including inhibition of TOR.

Although the anti-proliferative effects of rapamycin may be achieved through systemic use, superior results may be achieved through the local delivery of the compound. Essentially, rapamycin works in the tissues, which are in proximity to the compound, and has diminished effect as the distance from the delivery device increases.

Any number of non-erodible polymers may be utilized in conjunction with the rapamycin. In one exemplary embodiment, the polymeric matrix comprises two layers. The base layer comprises a solution of poly(ethylene-co-vinylacetate) and polybutylmethacrylate. The rapamycin is incorporated into this base layer. The outer layer comprises only polybutylmethacrylate and acts as a diffusion barrier to prevent the rapamycin from eluting too quickly. The thickness of the outer layer or topcoat determines the rate at which the rapamycin elutes from the matrix. Essentially, the rapamycin elutes from the matrix by diffusion through the polymer matrix. Polymers are permeable, thereby allowing solids, liquids and gases to escape therefrom. The total thickness of the polymeric matrix is in the range from about one micron to about twenty microns or greater. It is important to note that primer layers and metal surface treatments may be utilized before the polymeric matrix is affixed to the medical device. For example, acid cleaning, alkaline (base) cleaning, salinization and parylene deposition may be used as part of the overall process described above.

Alternatively the rapamycin or other therapeutic agent may be incorporated into a film-forming polyfluoro copolymer comprising an amount of a first moiety selected from the group consisting of polymerized vinylidenefluoride and polymerized tetrafluoroethylene, and an amount of a second moiety other than the first moiety and which is copolymerized with the first moiety, thereby producing the polyfluoro copolymer, the second moiety being capable of providing toughness or elastomeric properties to the polyfluoro copolymer, wherein the relative amounts of the first moiety and the second moiety are effective to provide the coating and film produced therefrom with properties effective for use in treating implantable medical devices.

The present invention provides polymeric coatings comprising a polyfluoro copolymer and implantable medical devices, coated with a film of the polymeric coating in amounts effective to reduce thrombosis and/or restenosis when such stents are used in, for example, angioplasty procedures. As used herein, polyfluoro copolymers means those copolymers comprising an amount of a first moiety selected from the group consisting of polymerized vinylidenefluoride and polymerized tetrafluoroethylene, and an amount of a second moiety other than the first moiety and which is copolymerized with the first moiety to produce the polyfluoro copolymer, the second moiety being capable of providing toughness or elastomeric properties to the polyfluoro copolymer, wherein the relative amounts of the first moiety and the second moiety are effective to provide coatings and film made from such polyfluoro copolymers with properties effective for use in coating implantable medical devices.

The coatings may comprise pharmaceutical or therapeutic agents for reducing restenosis, inflammation, and/or thrombosis. Films prepared from certain polyfluoro copolymer coatings of the present invention provide the physical and mechanical properties required of conventional coated medical devices, even where maximum temperature, to which the device coatings and films are exposed, are limited to relatively low temperatures. This is particularly important when using the coating/film to deliver pharmaceutical/therapeutic agents or drugs that are heat sensitive, or when applying the coating onto temperature-sensitive devices such as catheters. When maximum exposure temperature is not an issue, for example, where heat-stable agents such as itraconazole are incorporated into the coatings, higher melting thermoplastic polyfluoro copolymers may be used and, if very high elongation and adhesion is required, elastomers may be used. If desired or required, the polyfluoro elastomers may be crosslinked by standard methods described in, e.g., Modern Fluoropolymers, (J. Shires ed.), John Wiley & Sons, New York, 1997, pp. 77-87.

The present invention comprises polyfluoro copolymers that provide improved biocompatible coatings or vehicles for medical devices. These coatings provide inert biocompatible surfaces to be in contact with body tissue of a mammal, for example, a human, sufficient to reduce restenosis, or thrombosis, or other undesirable reactions. While many reported coatings made from polyfluoro homopolymers are insoluble and/or require high heat, for example, greater than about one hundred twenty-five degrees centigrade, to obtain films with adequate physical and mechanical properties for use on implantable devices, or are not particularly tough or elastomeric, films prepared from the polyfluoro copolymers of the present invention provide adequate adhesion, toughness or elasticity, and resistance to cracking when formed on medical devices. In certain exemplary embodiments, this is the case even where the devices are subjected to relatively low maximum temperatures.

The polyfluoro copolymers used for coatings according to the present invention are preferably film-forming polymers that have molecular weight high enough so as not to be waxy or tacky. The polymers and films formed therefrom should preferably adhere to the stent and not be readily deformable after deposition on the stent as to be able to be displaced by hemodynamic stresses. The polymer molecular weight should preferably be high enough to provide sufficient toughness so that films comprising the polymers will not be rubbed off during handling or deployment of the stent. In certain exemplary embodiments the coating will not crack where expansion of the stent or other medical devices occurs.

Coatings of the present invention comprise polyfluoro copolymers, as defined hereinabove. The second moiety polymerized with the first moiety to prepare the polyfluoro copolymer may be selected from those polymerized, biocompatible monomers that would provide biocompatible polymers acceptable for implantation in a mammal, while maintaining sufficient elastomeric film properties for use on medical devices claimed herein. Such monomers include, without limitation, hexafluoropropylene (HFP), tetrafluoroethylene (TFE), vinylidenefluoride, 1-hydropentafluoropropylene, perfluoro(methyl vinyl ether), chlorotrifluoroethylene (CTFE), pentafluoropropene, trifluoroethylene, hexafluoroacetone and hexafluoroisobutylene.

Polyfluoro copolymers used in the present invention typically comprise vinylidinefluoride copolymerized with hexafluoropropylene, in the weight ratio in the range of from about fifty to about ninety-two weight percent vinylidinefluoride to about fifty to about eight weight percent HFP. Preferably, polyfluoro copolymers used in the present invention comprise from about fifty to about eighty-five weight percent vinylidinefluoride copolymerized with from about fifty to about fifteen weight percent HFP. More preferably, the polyfluoro copolymers will comprise from about fifty-five to about seventy weight percent vinylidineflyoride copolymerized with from about forty-five to about thirty weight percent HFP. Even more preferably, polyfluoro copolymers comprise from about fifty-five to about sixty-five weight percent vinylidinefluoride copolymerized with from about forty-five to about thirty-five weight percent HFP. Such polyfluoro copolymers are soluble, in varying degrees, in solvents such as dimethylacetamide (DMAc), tetrahydrofuran, dimethyl formamide, dimethyl sulfoxide and n-methyl pyrrolidone. Some are soluble in methylethylketone (MEK), acetone, methanol and other solvents commonly used in applying coatings to conventional implantable medical devices.

Conventional polyfluoro homopolymers are crystalline and difficult to apply as high quality films onto metal surfaces without exposing the coatings to relatively high temperatures that correspond to the melting temperature (Tm) of the polymer. The elevated temperature serves to provide films prepared from such PVDF homopolymer coatings that exhibit sufficient adhesion of the film to the device, while preferably maintaining sufficient flexibility to resist film cracking upon expansion/contraction of the coated medical device. Certain films and coatings according to the present invention provide these same physical and mechanical properties, or essentially the same properties, even when the maximum temperatures to which the coatings and films are exposed is less than about a maximum predetermined temperature. This is particularly important when the coatings/films comprise pharmaceutical or therapeutic agents or drugs that are heat sensitive, for example, subject to chemical or physical degradation or other heat-induced negative affects, or when coating heat sensitive substrates of medical devices, for example, subject to heat-induced compositional or structural degradation.

Depending on the particular device upon which the coatings and films of the present invention are to be applied and the particular use/result required of the device, polyfluoro copolymers used to prepare such devices may be crystalline, semi-crystalline or amorphous.

Where devices have no restrictions or limitations with respect to exposure of same to elevated temperatures, crystalline polyfluoro copolymers may be employed. Crystalline polyfluoro copolymers tend to resist the tendency to flow under applied stress or gravity when exposed to temperatures above their glass transition (Tg) temperatures. Crystalline polyfluoro copolymers provide tougher coatings and films than their fully amorphous counterparts. In addition, crystalline polymers are more lubricious and more easily handled through crimping and transfer processes used to mount self-expanding stents, for example, nitinol stents.

Semi-crystalline and amorphous polyfluoro copolymers are advantageous where exposure to elevated temperatures is an issue, for example, where heat-sensitive pharmaceutical or therapeutic agents are incorporated into the coatings and films, or where device design, structure and/or use preclude exposure to such elevated temperatures. Semi-crystalline polyfluoro copolymer elastomers comprising relatively high levels, for example, from about thirty to about forty-five weight percent of the second moiety, for example, HFP, copolymerized with the first moiety, for example, VDF, have the advantage of reduced coefficient of friction and self-blocking relative to amorphous polyfluoro copolymer elastomers. Such characteristics may be of significant value when processing, packaging and delivering medical devices coated with such polyfluoro copolymers. In addition, such polyfluoro copolymer elastomers comprising such relatively high content of the second moiety serves to control the solubility of certain agents, for example, rapamycin, in the polymer and therefore controls permeability of the agent through the matrix.

Polyfluoro copolymers utilized in the present inventions may be prepared by various known polymerization methods. For example, high pressure, free-radical, semi-continuous emulsion polymerization techniques such as those disclosed in Fluoroelastomers-dependence of relaxation phenomena on compositions, POLYMER 30, 2180, 1989, by Ajroldi, et al., may be employed to prepare amorphous polyfluoro copolymers, some of which may be elastomers. In addition, free-radical batch emulsion polymerization techniques disclosed herein may be used to obtain polymers that are semi-crystalline, even where relatively high levels of the second moiety are included.

Suitable biocompatible metals include, but are not limited to, stainless steel, tantalum, titanium alloys (including nitinol), and cobalt alloys (including cobalt-chromium nickel alloys). Suitable nonmetallic biocompatible materials include, but are not limited to, polyamides, polyolefins (i.e. polypropylene, polyethylene etc.), nonabsorbable polyesters (i.e. polyethylene terephthalate), and bioabsorbable aliphatic polyesters (i.e. homopolymers and copolymers of lactic acid, glycolic acid, lactide, glycolide, para-dioxanone, trimethylene carbonate, ε-caprolactone, and blends thereof).

The polyfluoro copolymer coatings may be applied in one or more coating steps, depending on the amount of polyfluoro copolymer to be applied. Different polyfluoro copolymers may be used for different layers in the stent coating. In fact, in certain exemplary embodiments, it is highly advantageous to use a diluted first coating solution comprising a polyfluoro copolymer as a primer to promote adhesion of a subsequent polyfluoro copolymer coating layer that may include pharmaceutically active materials. The individual coatings may be prepared from different polyfluoro copolymers.

Additionally, a top coating may be applied to delay release of the pharmaceutical agent, or they could be used as the matrix for the delivery of a different pharmaceutically active material. Layering of coatings may be used to stage release of the drug or to control release of different agents placed in different layers.

Blends of polyfluoro copolymers may also be used to control the release rate of different agents or to provide a desirable balance of coating properties, i.e. elasticity, toughness, etc., and drug delivery characteristics, for example, release profile. Polyfluoro copolymers with different solubilities in solvents may be used to build up different polymer layers that may be used to deliver different drugs or to control the release profile of a drug. For example, polyfluoro copolymers comprising 85.5/14.5 (wt/wt) of poly(vinylidinefluoride/HFP) and 60.6/39.4 (wt/wt) of poly(vinylidinefluoride /HFP) are both soluble in DMAc. However, only the 60.6/39.4 PVDF polyfluoro copolymer is soluble in methanol. So, a first layer of the 85.5/14.5 PVDF polyfluoro copolymer comprising a drug could be over coated with a topcoat of the 60.6/39.4 PVDF polyfluoro copolymer made with the methanol solvent. The top coating may be used to delay the drug delivery of the drug contained in the first layer. Alternately, the second layer could comprise a different drug to provide for sequential drug delivery. Multiple layers of different drugs could be provided by alternating layers of first one polyfluoro copolymer, then the other. As will be readily appreciated by those skilled in the art, numerous layering approaches may be used to provide the desired drug delivery.

Coatings may be formulated by mixing one or more therapeutic agents with the coating polyfluoro copolymers in a coating mixture. The therapeutic agent may be present as a liquid, a finely divided solid, or any other appropriate physical form. Optionally, the coating mixture may include one or more additives, for example, nontoxic auxiliary substances such as diluents, carriers, excipients, stabilizers or the like. Other suitable additives may be formulated with the polymer and pharmaceutically active agent or compound. For example, a hydrophilic polymer may be added to a biocompatible hydrophobic coating to modify the release profile, or a hydrophobic polymer may be added to a hydrophilic coating to modify the release profile. One example would be adding a hydrophilic polymer selected from the group consisting of polyethylene oxide, polyvinyl pyrrolidone, polyethylene glycol, carboxylmethyl cellulose, and hydroxymethyl cellulose to a polyfluoro copolymer coating to modify the release profile. Appropriate relative amounts may be determined by monitoring the *in vitro* and/or *in vivo* release profiles for the therapeutic agents.

The best conditions for the coating application are when the polyfluoro copolymer and pharmaceutic agent have a common solvent. This provides a wet coating that is a true solution. Less desirable, yet still usable, are coatings that contain the pharmaceutical agent as a solid dispersion in a solution of the polymer in solvent. Under the dispersion conditions, care must be taken to ensure that the particle size of the dispersed pharmaceutical powder, both the primary powder size and its aggregates and agglomerates, is small enough not to cause an irregular coating surface or to clog the slots of the stent that need to remain essentially free of coating. In cases where a dispersion is applied to the stent and the smoothness of the coating film surface requires improvement, or to be ensured that all particles of the drug are fully encapsulated in the polymer, or in cases where the release rate of the drug is to be slowed, a clear (polyfluoro copolymer only) topcoat of the same polyfluoro copolymer used to provide sustained release of the drug or another polyfluoro copolymer that further restricts the diffusion of the drug out of the coating may be applied. The topcoat may be applied by dip coating with mandrel to clear the slots. This method is disclosed in United States Patent No. 6,153,252. Other methods for applying the topcoat include spin coating and spray coating. Dip coating of the topcoat can be problematic if the drug is very soluble in the coating solvent, which swells the polyfluoro copolymer, and the clear coating solution acts as a zero concentration sink and redissolves previously deposited drug. The time spent in the dip bath may need to be limited so that the drug is not extracted out into the drug-free bath. Drying should be rapid so that the previously deposited drug does not completely diffuse into the topcoat.

The amount of therapeutic agent will be dependent upon the particular drug employed and medical condition being treated. Typically, the amount of drug represents about 0.001 percent to about seventy percent of the total coating weight, more typically about 0.001 percent to about sixty percent of the total coating weight. It is possible that the drug may represent as little as 0.0001 percent to the total coating weight.

The quantity and type of polyfluoro copolymers employed in the coating film comprising the pharmaceutic agent will vary depending on the release profile desired and the amount of drug employed. The product may contain blends of the same or different polyfluoro copolymers having different molecular weights to provide the desired release profile or consistency to a given formulation.

Polyfluoro copolymers may release dispersed drug by diffusion. This can result in prolonged delivery (over, say approximately one to two-thousand hours, preferably two to eight-hundred hours) of effective amounts (0.001 µg/cm²-min to 1000 µg/cm²-min) of the drug. The dosage may be tailored to the subject being treated, the severity of the affliction, the judgment of the prescribing physician, and the like.

Individual formulations of drugs and polyfluoro copolymers may be tested in appropriate *in vitro* and *in vivo* models to achieve the desired drug release profiles. For example, a drug could be formulated with a polyfluoro copolymer, or blend of polyfluoro copolymers, coated onto a stent and placed in an agitated or circulating fluid system, for example, twenty-five percent ethanol in water. Samples of the circulating fluid could be taken to determine the release profile (such as by HPLC, UV analysis or use of radiotagged molecules). The release of a pharmaceutical compound from a stent coating into the interior wall of a lumen could be modeled in appropriate animal system. The drug release profile could then be monitored by appropriate means such as, by taking samples at specific times and assaying the samples for drug concentration (using HPLC to detect drug concentration). Thrombus formation can be modeled in animal models using the In-platelet imaging methods described by Hanson and Harker, Proc. Natl. Acad. Sci. USA 85:3184-3188 (1988). Following this or similar procedures, those skilled in the art will be able to formulate a variety of stent coating formulations.

While not a requirement of the present invention, the coatings and films may be crosslinked once applied to the medical devices. Crosslinking may be affected by any of the known crosslinking mechanisms, such as chemical, heat or light. In addition, crosslinking initiators and promoters may be used where applicable and appropriate. In those exemplary embodiments utilizing crosslinked films comprising pharmaceutical agents, curing may affect the rate at which the drug diffuses from the coating. Crosslinked polyfluoro copolymers films and coatings of the present invention also may be used without drug to modify the surface of implantable medical devices.

Other drugs, agents or compounds that may be particularly useful for use in combination with vascular filters include streptokinase, reteplase, temecteplase, urokinase, lanoteplase, staphylokinase, tissue plasminogen activator. In addition, compounds that enzymatically degrade emboli may be particularly useful.

Although shown and described is what is believed to be the most practical and preferred embodiments, it is apparent that departures from specific designs and methods described and shown will suggest themselves to those skilled in the art and may be used without departing from the spirit and scope of the invention. The present invention is not restricted to the particular constructions described and illustrated, but should be constructed to cohere with all modifications that may fall within the scope of the appended claims.

## Claims

1. A removable percutaneous vascular filter system for blocking micro-and macro-emboli in a vessel while allowing continued perfusion of blood, comprising:
a Guidewire (4000) having distal and proximal ends;
a filter (4002) including a non-metallic, porous, flexible filter membrane (4006) having a distal portion and a proximal free portion and a filter membrane support structure extending from the flexible filter membrane distal portion to at least the flexible filter membrane proximal portion, wherein the filter membrane distal portion is pivotally attached to the guidewire (4000) near the distal end of the guidewire (4000) and wherein the filter membrane proximal free end portion is substantially parallel to the guidewire (4000) in its collapsed state;
deploying means operatively connected to the filter (4002) to cause the filter membrane proximal free end portion to move from a position substantially parallel to the guidewire (4000) to a position removed from the longitudinal axis of the guidewire to cause the flexible filter membrane (4006) to form a substantially conical shape to form a generally sealing relationship with the wall of the vessel;
a biocompatible vehicle affixed to the filter membrane (4006); and
at least one agent in therapeutic dosages incorporated into the biocompatible vehicle for the treatment of a disease condition.

2. The removable percutaneous vascular filter system according to Claim 1, wherein the biocompatible vehicle comprises a polymeric matrix.

3. The removable percutaneous vascular filter system according to Claim 2, wherein the polymeric matrix comprises poly(ethylene-co-vinylacetate) and polybutylmethacrylate.

4. The removable percutaneous vascular filter system according to Claim 3, wherein the polymeric matrix comprises first and second layers, the first layer making contact with the filter membrane (4006) and comprising a solution of poly(ethylene-co-vinylacetate) and polybutylmethacrylate and the second layer comprising polybutylmethacrylate.

5. The removable percutaneous vascular filter system according to Claim 4, wherein the at least one agent is incorporated into the first layer.

6. The removable percutaneous vascular filter system according to Claim 1, wherein the biocompatible vehicle comprises a polyfluoro copolymer comprising polymerized residue of a first moiety selected from the group consisting of vinylidenefluoride and tetrafluoroethylene, and polymerized residue of a second moiety other than the first moiety and which is copolymerized with the first moiety, thereby producing the polyfluoro copolymer, wherein the relative amounts of the polymerized residue of the first moiety and the polymerized residue of the second moiety are effective to produce the biocompatible vehicle with properties effective for use in coating implantable medical devices when the coated medical device is subjected to a predetermined maximum temperature, and a solvent in which the polyfluoro copolymer is substantially soluble.

7. The removable percutaneous vascular filter system according to Claim 6, wherein the polyfluoro copolymer comprises from about fifty to about ninety-two weight percent of the polymerized residue of the first moiety copolymerized with from about fifty to about eight weight percent of the polymerized residue of the second moiety.

8. The removable percutaneous vascular filter system according to Claim 6, wherein the polyfluoro copolymer comprises from about fifty to about eighty-five weight percent of the polymerized residue of vinylidenefluoride copolymerized with from about fifty to about fifteen weight percent of the polymerized residue of the second moiety.

9. The removable percutaneous vascular filter system according to Claim 6, wherein said copolymer comprises from about fifty-five to about sixty-five weight percent of the polymerized residue of the vinylidenefluoride copolymerized with from about forty-five to about thirty-five weight percent of the polymerized residue of the second moiety.

10. The removable percutaneous vascular filter system according to Claim 6, wherein the second moiety is selected from the group consisting of hexafluoropropylene, tetrafluoroethylene, vinylidenefluoride, 1-hydropentafluoropropylene, perfluoro (methyl vinyl ether), chlorotrifluoroethylene, pentafluoropropene, trifluoroethylene, hexafluoroacetone and hexafluoroisobutylene.

11. The removable percutaneous vascular filter system according to Claim 6, wherein the second moiety is hexafluoropropylene.

12. The removable percutaneous vascular filter system according to Claim 1, wherein the at least one agent comprises an anti-proliferative.

13. The removable percutaneous vascular filter system according to Claim 1, wherein the at least one agent comprises an anti-inflammatory.

14. The removable percutaneous vascular filter system according to Claim 1, wherein the at least one agent comprises an anti-coagulant.

15. The removable percutaneous vascular filter system according to Claim 1, wherein the at least one agent comprises rapamycin.

16. The removable percutaneous vascular filter system according to Claim 1, wherein the at least one agent comprises heparin.

17. The removable percutaneous vascular filter system according to Claim 1, wherein the at least one agent comprises dexamethasone.

## Patentansprüche

1. Ein entfernbares perkutanes Gefäßfiltersystem zum Blockieren von Mikro- und Makroembolien in einem Gefäß bei gleichzeitigem Ermöglichen fortgesetzter Perfusion von Blut, umfassend:
einen Führungsdraht (4000), der distale und proximale Enden aufweist;
einen Filter (4002), der eine nicht-metallische, poröse, flexible Filtermembran (4006) aufweist, die einen distalen Abschnitt und einen proximalen freien Abschnitt und eine Filtermembranstützstruktur aufweist, die sich von dem distalen Abschnitt der flexiblen Filtermembran bis wenigstens zu dem proximalen Abschnitt der flexiblen Filtermembran erstreckt, wobei der distale Abschnitt der Filtermembran an dem Führungsdraht (4000) nahe dem distalen Ende des Führungsdrahts (4000) schwenkbar befestigt ist, und wobei der proximale freie Endabschnitt der Filtermembran im wesentlichen parallel zu dem Führungsdraht (4000) in seinem zusammengefalteten Zustand ist;
Einsetzmittel, die mit dem Filter (4002) in Wirkverbindung stehen, um den proximalen freien Endabschnitt der Filtermembran zu veranlassen, sich von einer Position, die im wesentlichen parallel zu dem Führungsdraht (4000) liegt, zu einer Position zu bewegen, die von der longitudinalen Achse des Führungsdrahts entfernt liegt, um die flexible Filtermembran (4006) zu veranlassen, eine im wesentlichen kegelförmige Form zu bilden, um eine allgemein abdichtende Beziehung zu der Wand des Gefäßes zu bilden;
einen biokompatiblen Trägerstoff, der an der Filtermembran (4006) befestigt ist; und
wenigstens ein Agens in therapeutischer Dosierung, das in den biokompatiblen Trägerstoff zur Behandlung eines Krankheitszustands aufgenommen ist.

2. Entfernbares perkutanes Gefäßfiltersystem nach Anspruch 1, **dadurch gekennzeichnet, dass** der biokompatible Trägerstoff eine Polymermatrix umfasst.

3. Entfernbares perkutanes Gefäßfiltersystem nach Anspruch 2, **dadurch gekennzeichnet, dass** die Polymermatrix Poly(ethylen-co-vinylacetat) und Polybutylmethacrylat umfasst.

4. Entfernbares perkutanes Gefäßfiltersystem nach Anspruch 3, **dadurch gekennzeichnet, dass** die Polymermatrix erste und zweite Schichten umfasst, wobei die erste Schicht Kontakt mit der Filtermembran (4006) aufnimmt und eine Lösung von Poly(ethylen-co-vinylacetat) und Polybutylmethacrylat aufweist, und die zweite Schicht Polybutylmethacrylat aufweist.

5. Entfernbares perkutanes Gefäßfiltersystem nach Anspruch 4, **dadurch gekennzeichnet, dass** das wenigstens eine Agens in die erste Schicht aufgenommen ist.

6. Entfernbares perkutanes Gefäßfiltersystem nach Anspruch 1, **dadurch gekennzeichnet, dass** der biokompatible Trägerstoff ein Polyfluorcopolymer aufweist, das polymerisierten Rest eines ersten Teils, der aus der Gruppe bestehend aus Vinylidenfluorid und Tetrafluorethylen ausgewählt ist, und polymerisierten Rest eines zweiten Teils aufweist, der sich von dem ersten Teil unterscheidet und mit dem ersten Teil copolymerisiert ist und dabei das Polyfluorcopolymer erzeugt, wobei die relativen Mengen des polymerisierten Restes des ersten Teils und des polymerisierten Restes des zweiten Teils bewirken, dass der biokompatible Trägerstoff mit Eigenschaften erzeugt wird, die eine Verwendung zum Beschichten implantierbarer medizinischer Einrichtungen bewirken, wenn die beschichteten medizinischen Einrichtungen einer vorbestimmten maximalen Temperatur unterworfen werden, sowie ein Lösungsmittel, in welchem das Polyfluorcopolymer im wesentlichen lösbar ist.

7. Entfernbares perkutanes Gefäßfiltersystem nach Anspruch 6, **dadurch gekennzeichnet, dass** das Polyfluorcopolymer etwa 50 bis etwa 92 Gewichtsprozent der polymerisierten Reste des ersten Teils umfasst, der mit etwa 50 bis etwa 80 Gewichtsprozent der polymerisierten Reste des zweiten Teils copolymerisiert ist.

8. Entfernbares perkutantes Gefäßfilterfiltersystem nach Anspruch 6, **dadurch gekennzeichnet, dass** das Polyfluorcopolymer etwa 50 bis etwa 85 Gewichtsprozent des polymerisierten Restes von Vinylidenfluorid umfasst, der mit etwa 50 bis etwa 15 Gewichtsprozent des polymerisierten Restes des zweiten Teils copolymerisiert ist.

9. Entfernbares perkutanes Gefäßfiltersystem nach Anspruch 6, **dadurch gekennzeichnet, dass** das Copolymer etwa 55 bis etwa 65 Gew.-% des polymerisierten Restes des Vinylidenfluorids aufweist, das mit etwa 45 bis etwa 35 Gewichtsprozent des polymerisierten Restes des zweiten Teils copolymerisiert ist.

10. Entfernbares perkutanes Gefäßfiltersystem nach Anspruch 6, **dadurch gekennzeichnet, dass** der zweite Teil aus einer Gruppe bestehend aus Hexafluorpropylen, Tetrafluorethylen, Vinylidenfluorid, 1-Hydropentafluorpropylen, Perfluor(methylvinylether), Chlorotrifluorethylen, Pentafluorpropen, Trifluorethylen, Hexafluoraceton und Hexafluoroisobutylen ausgewählt ist.

11. Entfernbares perkutanes Gefäßfiltersystem nach Anspruch 6, **dadurch gekennzeichnet, dass** der zweite Teil Hexafluorpropylen ist.

12. Entfernbares perkutanes Gefäßfiltersystem nach Anspruch 1, **dadurch gekennzeichnet, dass** das wenigstens eine Agens ein wachstumhemmendes Mittel umfasst.

13. Entfernbares perkutanes Gefäßfiltersystem nach Anspruch 1, **dadurch gekennzeichnet, dass** das wenigstens eine Agens ein entzündungshemmendes Mittel aufweist.

14. Entfernbares perkutanes Gefäßfiltersystem nach Anspruch 1, **dadurch gekennzeichnet, dass** das wenigstens eine Agens ein gerinnungshemmendes Mittel aufweist.

15. Entfernbares perkutanes Gefäßfiltersystem nach Anspruch 1, **dadurch gekennzeichnet, dass** das wenigstens eine Agens Rapamycin aufweist.

16. Entfernbares perkutanes Gefäßfiltersystem nach Anspruch 1, **dadurch gekennzeichnet, dass** das wenigstens eine Agens Heparin aufweist.

17. Entfernbares perkutanes Gefäßfiltersystem nach Anspruch 1, **dadurch gekennzeichnet, dass** das wenigstens eine Agens Dexamethason aufweist.

## Revendications

1. Système de filtre vasculaire percutané amovible destiné à bloquer des micro-emboles et des macro-emboles dans un vaisseau tout en permettant une irrigation continue du sang, comprenant :
➢ un fil de guidage (4000) qui présente des extrémités distale et proximale ;
➢ un filtre (4002) qui comprend une membrane de filtrage (4006) non métallique, poreuse et souple qui présente une partie distale et une partie proximale libre et une structure de support de membrane de filtrage qui s'étend à partir de la partie distale de la membrane de filtrage souple jusqu'au moins la partie proximale de la membrane de filtrage souple, dans lequel la partie distale de la membrane de filtrage est fixée de manière pivotante au fil de guidage (4000) à proximité de l'extrémité distale du fil de guidage (4000) et dans lequel la partie d'extrémité libre proximale de la membrane de filtrage est sensiblement parallèle au fil de guidage (4000) dans son état effondré ;
➢ des moyens de déploiement reliés de façon opérationnelle au filtre (4002) de manière à provoquer le déplacement de la partie d'extrémité libre proximale de la membrane de filtrage, à partir d'une position sensiblement parallèle au fil de guidage (4000) jusqu'à une position retirée de l'axe longitudinal du fil de guidage de manière à ce que la membrane de filtrage souple (4006) présente une forme sensiblement conique de façon à établir une relation en général d'étanchéité avec la paroi du vaisseau ;
➢ un véhicule biocompatible fixé à la membrane de filtrage (4006) ; et
➢ au moins un agent en dosages thérapeutiques incorporé dans le véhicule biocompatible pour le traitement d'un état de maladie.

2. Système de filtre vasculaire percutané amovible selon la revendication 1, dans lequel le véhicule biocompatible comprend une matrice polymère.

3. Système de filtre vasculaire percutané amovible selon la revendication 2, dans lequel la matrice polymère comprend un poly(éthylène-co-vinylacétate) et un polybutylméthacrylate.

4. Système de filtre vasculaire percutané amovible selon la revendication 3, dans lequel la matrice polymère comprend des première et seconde couches, la première couche établissant un contact avec la membrane de filtrage (4006) et comprenant une solution de poly-(éthylène-co-vinylacétate) et de polybutylméthacrylate et la seconde couche comprenant du polybutylméthacrylate.

5. Système de filtre vasculaire percutané amovible selon la revendication 4, dans lequel l'au moins un agent est incorporé dans la première couche.

6. Système de filtre vasculaire percutané amovible selon la revendication 1, dans lequel le véhicule biocompatible comprend un copolymère polyfluoré qui comprend un résidu polymérisé d'un premier groupe caractéristique choisi dans le groupe constitué par le fluorure de vinylidène et le tétrafluoroéthylène, et un résidu polymérisé d'un second groupe caractéristique différent du premier groupe caractéristique et qui est copolymerisé avec le premier groupe caractéristique, en produisant de ce fait le copolymère polyfluoré, dans lequel les quantités relatives de résidu polymérisé du premier groupe caractéristique et de résidu polymérisé du second caractéristique sont efficaces pour produire le véhicule biocompatible avec des propriétés efficaces pour une utilisation d'enduction de dispositifs médicaux pouvant être implantés lorsque le dispositif médical enduit est soumis à une température maximale prédéterminée, et à un solvant dans lequel le copolymère polyfluoré est sensiblement soluble.

7. Système de filtre vasculaire percutané amovible selon la revendication 6, dans lequel le copolymère polyfluoré comprend entre environ cinquante et environ quatre-vingt-douze pour cent en poids de résidu polymérisé du premier groupe caractéristique copolymerisé avec entre environ cinquante et environ huit pour cent en poids de résidu polymérisé du second groupe caractéristique.

8. Système de filtre vasculaire percutané amovible selon la revendication 6, dans lequel le copolymère polyfluoré comprend entre environ cinquante et environ quatre-vingt-cinq pour cent en poids de résidu polymérisé de fluorure de vinylidène copolymerisé avec entre environ cinquante et environ quinze pour cent en poids de résidu polymérisé du second groupe caractéristique.

9. Système de filtre vasculaire percutané amovible selon la revendication 6, dans lequel ledit copolymère comprend entre environ cinquante-cinq et environ soixante-cinq pour cent en poids de résidu polymérisé de fluorure de vinylidène copolymerisé avec entre environ quarante-cinq et environ trente-cinq pour cent en poids de résidu polymérisé du second groupe caractéristique.

10. Système de filtre vasculaire percutané amovible selon la revendication 6, dans lequel le second groupe caractéristique est sélectionné dans le groupe constitué par l'hexafluoropropylène, le tétrafluoroéthylène, le fluorure de vinylidène, le 1-hydropentafluoropropylène, l' (éther de vinyle - méthyle) perfluoré, le chlorotrifluoroéthylène, le pentafluoropropène, le trifluoroéthylène, l'hexafluoroacétone et l'hexafluoroisobutylène.

11. Système de filtre vasculaire percutané amovible selon la revendication 6, dans lequel le second groupe caractéristique est l'hexafluoropropylène.

12. Système de filtre vasculaire percutané amovible selon la revendication 1,dans lequel l'au moins un agent comprend un anti-proliférant.

13. Système de filtre vasculaire percutané amovible selon la revendication 1,dans lequel l'au moins un agent comprend un anti-inflammatoire.

14. Système de filtre vasculaire percutané amovible selon la revendication 1, dans lequel l'au moins un agent comprend un anti-coagulant.

15. Système de filtre vasculaire percutané amovible selon la revendication 1, dans lequel l'au moins un agent comprend de la rapamycine.

16. Système de filtre vasculaire percutané amovible selon la revendication 1, dans lequel l'au moins un agent comprend de l'héparine.

17. Système de filtre vasculaire percutané amovible selon la revendication 1, dans lequel l'au moins un agent comprend de la dexaméthasone.
